# FASCICULE DE BREVET EUROPEEN

(11) **EP 3 361 962 B1**
(45) Date de publication et mention de la délivrance du brevet: **25.12.2019**
(21) Numéro de dépôt: 16806211.5
(22) Date de dépôt: 14.10.2016
(51) Int. Cl.: A61B 17/12

(54) **BALLONNET GONFLABLE À USAGE MÉDICAL**
AUFBLASBARER BALLON FÜR MEDIZINISCHE ZWECKE
INFLATABLE BALLOON FOR MEDICAL USE

(30) Priorité: 16.10.2015 FR 1559917
(43) Date de publication de la demande: 22.08.2018
(73) Titulaire: Dianosic, 92240 Malakoff (FR); Université de Strasbourg, 67081 Strasbourg cedex (FR); Institut National de la Santé et de la Recherche Médicale, 75654 Paris cedex 13 (FR); Hopitaux Universitaires de Strasbourg, 67091 Strasbourg cedex (FR)
(72) Inventeur: DEBRY,Christian, 75015 Paris (FR); AUGUSTIN, Marc, 75015 Paris (FR)
(74) Mandataire: Bonnet, Michel
(86) Numéro de dépôt international: PCT/FR2016/052661
(87) Numéro de publication internationale: WO 2017/064437

(56) Documents cités:
- WO-A1-96/39218
- DE-U1- 29 923 582
- US-A1- 2015 005 805

## Description

La présente invention concerne un ballonnet gonflable à usage médical, destiné à être inséré dégonflé dans une cavité de corps humain ou animal puis gonflé à l'intérieur de cette cavité en vue de faire pression contre les parois de cette cavité.

En otorhinolaryngologie par exemple, un ballonnet de ce type est inséré dans la cavité nasale d'un patient, puis gonflé une fois correctement positionné à l'aide d'un fluide tel que de l'air ou de l'eau gélifiée. Une application courante de ce type de ballonnet est le traitement d'hémorragies par pression contre les parois internes de la cavité nasale.

L'invention s'applique plus particulièrement à un ballonnet à usage médical comportant :
- un corps de ballonnet dont la paroi est en matériau souple gonflable, de forme générale allongée présentant une extrémité proximale de gonflage et une extrémité distale de positionnement en fond de cavité, et
- une ouverture de gonflage formée dans l'extrémité proximale, pour l'introduction d'un fluide dans le corps de ballonnet de manière à le gonfler sous pression.

Une difficulté dans l'utilisation de ce type de ballonnet est l'insertion et le positionnement du ballonnet dans la cavité, notamment lorsqu'il s'agit d'une insertion en cavité nasale.

Généralement, cette difficulté est surmontée par l'utilisation d'écarteurs rigides permettant une insertion facilitée du ballonnet ou se substituant même à lui, ou l'usage d'un guide rigide amovible solidaire du ballonnet. Enfin, le ballonnet est parfois monté autour d'un tube central, souvent traversant pour permettre la respiration du patient, ce tube central remplissant alors la fonction de guide pendant l'insertion du ballonnet dégonflé dans la cavité. De telles solutions sont bien connues et par exemple proposées dans des documents de brevets tels que US 2,265,387, US 3,516,407 et US 5,139,510.

Mais ces différentes solutions sont complexes, soit en termes de manipulation, soit en termes de conception, voire même les deux.

Document WO 96/39218 divulgue un dispositif gonflable de tamponnement nasal comportant une base flexible qui peut être introduite à travers la cavité nasale pour pénétrer dans le rhinopharynx. Un premier sac gonflable est fixé à la partie antérieure de la base et reste à l'intérieur de la cavité nasale, un deuxième sac gonflable est fixé à la partie postérieure de la base et pénètre dans le rhinopharynx. Ces sacs entourent entièrement la base et ils sont beaucoup plus grands que les parties correspondantes de la cavité nasale et du rhinopharynx. Le gonflage et le dégonflage des sacs sont commandés par deux sondes qui passent le long de la base.

Il peut ainsi être souhaité de prévoir un ballonnet gonflable à usage médical qui permette de s'affranchir d'au moins une partie des problèmes et contraintes précités.

Il est donc proposé un ballonnet gonflable à usage médical comportant les caractéristiques de la revendication indépendante.

Ainsi, le dispositif proposé est simple de conception puisque la semelle forme une portion de la paroi du corps de ballonnet. Il est en outre d'utilisation aisée puisque cette semelle de plus forte rigidité facilite l'insertion et le bon positionnement du ballonnet sur toute sa longueur dans la cavité.

De façon optionnelle, la semelle est une pièce rapportée contre la paroi du corps de ballonnet.

De façon optionnelle également, la semelle est venue de matière avec le reste de la paroi du corps de ballonnet mais présente une épaisseur sensiblement supérieure au reste de cette paroi.

De façon optionnelle également, un ballonnet gonflable à usage médical selon l'invention peut en outre comporter un renfort en extrémité distale dans le prolongement de la semelle, ce renfort distal présentant également une rigidité supérieure au reste de la paroi du corps de ballonnet et étant conformé de manière à empêcher, en coopération avec la semelle, tout allongement du ballonnet lors de son gonflage.

Une ouverture pour endoscope est en outre disposée dans l'extrémité proximale, cette ouverture pour endoscope étant destinée à l'introduction d'un endoscope dans le corps de ballonnet.

De façon optionnelle également, l'ouverture pour endoscope comporte une valve permettant l'introduction d'un endoscope tout en empêchant toute sortie de fluide.

L'ouverture pour endoscope débouche à l'intérieur du corps de ballonnet sur l'intérieur d'une chaussette allongée.

La chaussette allongée s'étendant à l'intérieur du corps de ballonnet présente une extrémité distale fermée non solidaire de la paroi du corps de ballonnet.

De façon optionnelle également, un ballonnet gonflable à usage médical selon l'invention peut s'utiliser en otorhinolaryngologie. Dans ce cas, le corps de ballonnet peut être préformé pour épouser les formes intérieures d'une cavité nasale de corps humain ou animal lorsqu'il est gonflé.

De façon optionnelle également, un ballonnet gonflable à usage médical selon l'invention peut en outre comporter une tige rigide d'introduction amovible s'étendant vers l'extérieur dans le prolongement d'un embout de l'ouverture de gonflage et un fil rigide amovible de mise en place s'étendant le long de la semelle ou dans cette dernière.

L'invention sera mieux comprise à l'aide de la description qui va suivre, donnée uniquement à titre d'exemple et faite en se référant aux dessins annexés dans lesquels :
- la figure 1 représente schématiquement la structure générale d'un ballonnet gonflable à usage médical, selon un mode de réalisation de l'invention,
- la figure 2 illustre un exemple de forme pour le ballonnet de la figure 1,
- les figures 3 et 4 illustrent des dispositions du ballonnet de la figure 1 dans une cavité nasale, avant et après gonflage, et
- les figures 5 et 6 représentent schématiquement la structure générale d'un ballonnet gonflable à usage médical, selon un autre mode de réalisation de l'invention, avant et après gonflage.

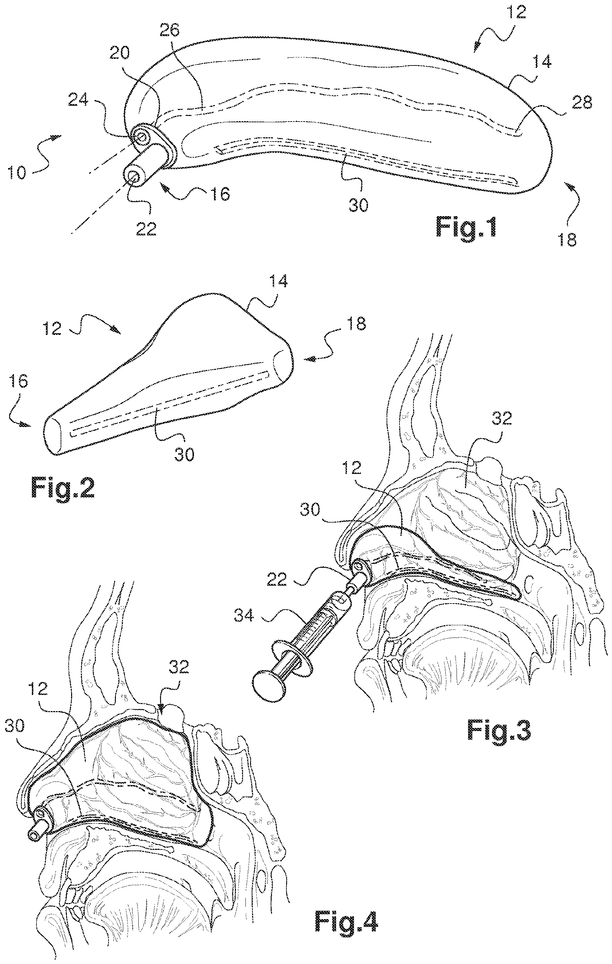

Le ballonnet gonflable 10 à usage médical représenté schématiquement sur la figure 1 comporte un corps de ballonnet 12 dont la paroi 14 est en matériau souple gonflable, par exemple en silicone ou tout matériau équivalent présentant ces propriétés. Il est destiné à être inséré dégonflé dans une cavité de corps humain ou animal puis gonflé à l'intérieur de cette cavité en vue de faire pression contre elle. Il est de forme générale allongée, présentant une extrémité proximale 16 de gonflage et une extrémité distale 18 de positionnement en fond de cavité. L'extérieur de la paroi 14 est avantageusement enduit d'un produit glissant et non agressif pour les tissus biologiques de la cavité.

L'extrémité proximale 16 comporte un support rigide 20 qui présente une première ouverture et un embout de gonflage 22 menant à cette première ouverture. Cet embout 22 facilite l'introduction d'une seringue pour gonfler le corps de ballonnet 12 sous pression à l'aide d'un fluide tel que de l'air, de l'eau gélifiée, ou tout autre fluide approprié. Il peut être muni d'une valve, ou de tout système approprié pour le gonflage, de façon à permettre une injection ou une aspiration de fluide sans qu'il n'y ait de fuites lorsque l'on enlève la seringue ou le dispositif de gonflage.

Le support rigide 20 présente en outre une seconde ouverture 24 destinée à l'introduction d'un endoscope dans le corps de ballonnet 12. Cette seconde ouverture 24 se présente par exemple sous la forme d'une valve ou de tout système permettant l'introduction d'un endoscope tout en empêchant toute sortie de fluide introduit dans le corps de ballonnet 12.

Dans ce cas, il est avantageux, voire nécessaire que le fluide soit le plus transparent ou translucide possible pour une meilleure observation de l'environnement par l'endoscope à l'intérieur du ballonnet 10.

Dans ce cas également, le ballonnet 10 comporte en outre une chaussette allongée 26 s'étendant à l'intérieur du corps de ballonnet 12 et présentant une extrémité distale fermée 28 non solidaire de la paroi 14 du corps de ballonnet 12. Une chaussette allongée doit être comprise comme un capuchon allongé, de forme complémentaire à celle de l'endoscope, à l'intérieur duquel débouche la seconde ouverture 24. Elle permet l'introduction de l'endoscope sans contact de ce dernier avec le fluide présent dans le corps de ballonnet 12. Elle est avantageusement formée dans un matériau souple éventuellement élastique, par exemple en silicone ou tout matériau de même nature.

Conformément à l'invention, la paroi 14 du corps de ballonnet 12 présente localement une portion allongée 30, dite semelle, de plus forte rigidité que le reste de la paroi 14, cette semelle 30 s'étendant de l'extrémité proximale 16 jusqu'à l'extrémité distale 18. Elle forme la base du corps de ballonnet 12, facilitant l'introduction et le maintien du ballonnet 10 dans la cavité souhaitée même lorsque celui-ci est dégonflé. Par ailleurs, elle remplit une fonction de guide lors du gonflage du ballonnet 10, en lui donnant une direction précontrainte.

En pratique la semelle 30 peut être une pièce rapportée contre la paroi 14 du corps de ballonnet 12, constituée de tout matériau assurant la fonction de rigidité voulue. Ce matériau peut être choisi de façon non limitative parmi des polymères tels que du polychlorure de vinyle, du polysiloxane, du polyuréthane, du polycarbonate polyéthylène, du polyméthacrylate de méthyle, du polytéréphtalate d'éthylène, ou parmi des polymères fluorés tels que du polytétrafluoroéthylène ou du polychlorotrifluoroéthylène. De façon alternative, elle peut être venue de matière avec le reste de la paroi 14 du corps de ballonnet 12, mais elle présente alors une épaisseur sensiblement supérieure au reste de cette paroi 14 pour augmenter sa rigidité.

Comme illustré sur la figure 2, le ballonnet 10 peut être conçu pour un usage en otorhinolaryngologie. Dans ce cas, la paroi 14 du corps de ballonnet 12 peut être préformée pour épouser les formes intérieures d'une cavité nasale de corps humain ou animal en conformation gonflée.

La figure 3 illustre le positionnement du ballonnet 10 des figures 1 et 2 dans une cavité nasale 32, en conformation dégonflée. Grâce à la semelle 30 de rigidité supérieure au reste de la paroi 14 du corps de ballonnet 12, le ballonnet 10 est facilement introduit dans la cavité nasale 32. Une fois positionné, un fluide est introduit par l'embout 22 dans le corps de ballonnet 12 à l'aide d'une seringue 34. En procédant de la sorte, la pression à l'intérieur du corps de ballonnet est facilement contrôlée.

Cela permet le gonflage du ballonnet 10 jusqu'à obtention du résultat illustré sur la figure 4 selon lequel le corps de ballonnet 12 occupe tout l'espace intérieur de la cavité nasale 32 en épousant la forme de ses parois.

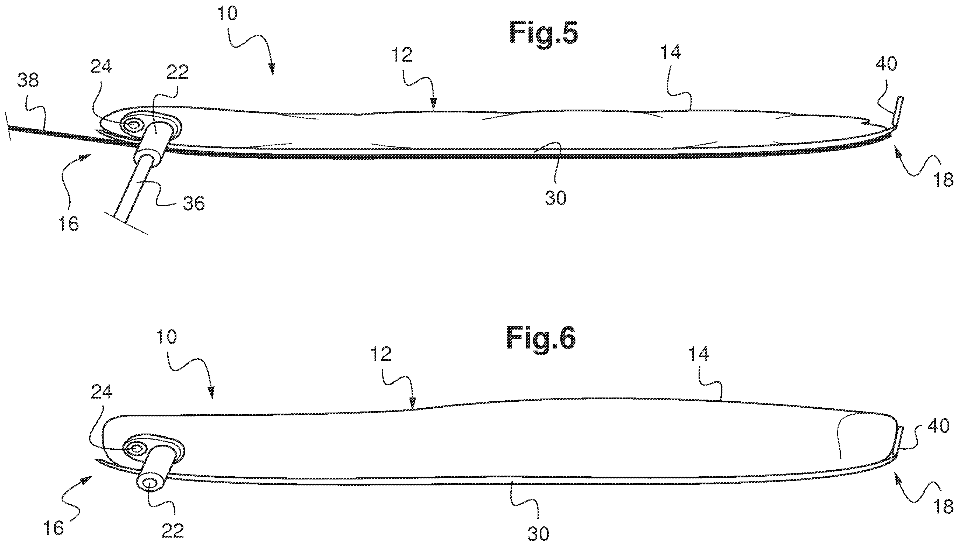

Selon un autre mode de réalisation illustré en coupe sur la figure 5, et en reprenant les mêmes références que précédemment pour les éléments inchangés, le ballonnet 10 comporte en outre une tige rigide 36 d'introduction amovible s'étendant vers l'extérieur dans le prolongement de l'embout 22. Cette tige rigide 36 permet de faciliter l'installation du ballonnet 10 dans la cavité et peut être rompue ou retirée lorsque cette installation est correctement réalisée. Le ballonnet 10 peut être gonflé à l'aide de la seringue 34 avant ou après cette manoeuvre.

Le ballonnet 10 de cet autre mode de réalisation comporte en outre un fil rigide amovible 38 de mise en place, s'étendant en renforcement de la semelle 30 le long de ou dans cette dernière. Il est par exemple conçu en alliage de nickel-titane, ce matériau possédant des propriétés intéressantes de mémoire de forme et d'élasticité.

Enfin, le ballonnet 10 de cet autre mode de réalisation comporte un renfort 40 en extrémité distale 18 dans le prolongement de la semelle 30. Ce renfort distal 40 présente, comme la semelle 30, une rigidité supérieure au reste de la paroi 14 du corps de ballonnet 12 et est conformé, par exemple selon un angle proche de 90° par rapport à la semelle 30, de manière à empêcher, en coopération avec cette dernière, tout allongement du ballonnet 10 lors de son gonflage. Il peut concrètement être constitué d'une pièce rapportée à l'extrémité distale de la semelle 30, constituée de tout matériau assurant la fonction de rigidité voulue. Ce matériau peut être choisi de façon non limitative parmi des polymères tels que du polychlorure de vinyle, du polysiloxane, du polyuréthane, du polycarbonate polyéthylène, du polyméthacrylate de méthyle, du polytéréphtalate d'éthylène, ou parmi des polymères fluorés tels que du polytétrafluoroéthylène ou du polychlorotrifluoroéthylène.

Le ballonnet 10 de cet autre mode de réalisation est représenté en conformation dégonflée sur la figure 5 et en conformation gonflée, avec retrait de la tige rigide 36 et du fil rigide amovible 38, sur la figure 6.

Il apparaît clairement qu'un ballonnet gonflable à usage médical tel que celui décrit précédemment permet une introduction et un positionnement aisés dans une cavité souhaitée de corps humain ou animal.

Compte tenu de sa structure simple, il est de conception et fabrication aisées, de sorte qu'il peut être à utilisation unique puis jeté.

Il est particulièrement adapté à des opérations médicales de chirurgies otorhinolaryngologiques, cervico-faciales ou neurologiques. Il peut aussi s'utiliser dans d'autres opérations chirurgicales, notamment en orthopédie ou chirurgie digestive. Sans même parler de chirurgie, il peut aussi s'utiliser en tant que simple dispositif de compression pour traiter toutes sortes d'épistaxis ou saignements de fosses nasales, de tels saignements se produisant par exemple couramment en consultation.

On notera par ailleurs que l'invention n'est pas limitée aux modes de réalisation décrits précédemment.

En particulier, les formes de la semelle 30 et du renfort distal 40 peuvent être adaptées à toutes les applications souhaitées. Elles sont donc potentiellement très diverses.

Il apparaîtra plus généralement à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications aux modes de réalisation exposés dans la présente description.

## Revendications

1. Ballonnet gonflable (10) à usage médical, destiné à être inséré dégonflé dans une cavité de corps humain ou animal puis gonflé à l'intérieur de cette cavité en vue de faire pression contre elle, comportant :
- un corps de ballonnet (12) dont la paroi (14) est en matériau souple gonflable, de forme générale allongée présentant une extrémité proximale (16) de gonflage et une extrémité distale (18) de positionnement en fond de cavité, la paroi (14) présentant en outre localement une portion allongée (30), dite semelle, de plus forte rigidité que le reste de la paroi (14), cette semelle s'étendant de l'extrémité proximale (16) jusqu'à l'extrémité distale (18),
- une ouverture de gonflage (22) formée dans l'extrémité proximale (16), pour l'introduction d'un fluide dans le corps de ballonnet (12) de manière à le gonfler sous pression, et
- une ouverture (24) pour endoscope en outre disposée dans l'extrémité proximale (16), cette ouverture (24) pour endoscope étant destinée à l'introduction d'un endoscope dans le corps de ballonnet (12),
**caractérisé en ce que** l'ouverture (24) pour endoscope débouche à l'intérieur du corps de ballonnet (12) sur l'intérieur d'une chaussette allongée (26) présentant une extrémité distale fermée (28) non solidaire de la paroi (14) du corps de ballonnet (12).

2. Ballonnet gonflable (10) à usage médical selon la revendication 1, dans lequel la semelle (30) est une pièce rapportée contre la paroi (14) du corps de ballonnet (12).

3. Ballonnet gonflable (10) à usage médical selon la revendication 1, dans lequel la semelle (30) est venue de matière avec le reste de la paroi (14) du corps de ballonnet (12) mais présente une épaisseur sensiblement supérieure au reste de cette paroi (14).

4. Ballonnet gonflable (10) à usage médical selon l'une quelconque des revendications 1 à 3, comportant en outre un renfort (40) en extrémité distale dans le prolongement de la semelle (30), ce renfort distal (40) présentant également une rigidité supérieure au reste de la paroi (14) du corps de ballonnet (12) et étant conformé de manière à empêcher, en coopération avec la semelle (30), tout allongement du ballonnet (10) lors de son gonflage.

5. Ballonnet gonflable (10) à usage médical selon l'une quelconque des revendications 1 à 4, dans lequel l'ouverture (24) pour endoscope comporte une valve permettant l'introduction d'un endoscope tout en empêchant toute sortie de fluide.

6. Ballonnet gonflable (10) à usage médical selon l'une quelconque des revendications 1 à 5, à utilisation en otorhinolaryngologie, dans lequel le corps de ballonnet (12) est préformé pour épouser les formes intérieures d'une cavité nasale (32) de corps humain ou animal lorsqu'il est gonflé.

7. Ballonnet gonflable (10) à usage médical selon l'une quelconque des revendications 1 à 6, comportant en outre une tige (36) d'introduction amovible s'étendant vers l'extérieur dans le prolongement d'un embout de l'ouverture de gonflage (22) et un fil amovible (38) de mise en place s'étendant le long de la semelle (30) ou dans cette dernière.

## Patentansprüche

1. Aufblasbarer Ballon (10) zur medizinischen Verwendung, der ausgelegt ist, um nicht aufgeblasen in einen Hohlraum des menschlichen oder tierischen Körpers eingeführt zu werden, dann im Inneren dieses Hohlraums aufgeblasen zu werden, um gegen denselben Druck auszuüben, umfassend:
- einen Ballonkörper (12), dessen Wand (14) aus einem aufblasbaren flexiblen Material mit einer im Allgemeinen länglichen Form ist, die ein proximales Ende (16) zum Aufblasen und ein distales Ende (18) zum Positionieren am Boden des Hohlraums aufweist, wobei die Wand (14) außerdem lokal einen verlängerten Abschnitt (30), bezeichnet als Sohle, mit einer größeren Steifigkeit als der Rest der Wand (14) aufweist, wobei sich diese Sohle vom proximalen Ende (16) bis zum distalen Ende (18) erstreckt,
- eine Aufblasöffnung (22), die im proximalen Ende (16) gebildet ist, zur Einführung eines Fluids in den Ballonkörper (12), um ihn unter Druck aufzublasen, und
- eine Öffnung (24) für ein Endoskop, die außerdem im proximalen Ende (16) angeordnet ist, wobei diese Öffnung (24) für ein Endoskop zur Einführung eines Endoskops in den Ballonkörper (12) ausgelegt ist,
**dadurch gekennzeichnet, dass** die Öffnung (24) für ein Endoskop im Inneren des Ballonkörpers (12) auf das Innere eines verlängerten Führungsrohrs (26) mündet, das ein geschlossenes distales Ende (28) aufweist, das nicht mit der Wand (14) des Ballonkörpers (12) fest verbunden ist.

2. Aufblasbarer Ballon (10) zur medizinischen Verwendung nach Anspruch 1, wobei die Sohle (30) ein Stück ist, das gegen die Wand (14) des Ballonkörpers (12) aufgesetzt ist.

3. Aufblasbarer Ballon (10) zur medizinischen Verwendung nach Anspruch 1, wobei die Sohle (30) aus dem gleichen Material wie der Rest der Wand (14) des Ballonkörpers (12) hergestellt ist, aber eine wesentlich größere Dicke als der Rest dieser Wand (14) aufweist.

4. Aufblasbarer Ballon (10) zur medizinischen Verwendung nach einem der Ansprüche 1 bis 3, umfassend außerdem eine Verstärkung (40) am distalen Ende in der Verlängerung der Sohle (30), wobei diese distale Verstärkung (40) auch eine größere Steifigkeit als der Rest der Wand (14) des Ballonkörpers (12) aufweist und ausgestaltet ist, um in Zusammenarbeit mit der Sohle (30) jede Verlängerung des Ballons (10) bei seinem Aufblasen zu verhindern.

5. Aufblasbarer Ballon (10) zur medizinischen Verwendung nach einem der Ansprüche 1 bis 4, wobei die Öffnung (24) für ein Endoskop ein Ventil umfasst, das die Einführung eines Endoskops ermöglicht und gleichzeitig jeden Austritt von Fluid verhindert.

6. Aufblasbarer Ballon (10) zur medizinischen Verwendung nach einem der Ansprüche 1 bis 5 zur Verwendung in der HNO-Medizin, wobei der Ballonkörper (12) vorgeformt ist, um sich den inneren Formen eines nasalen Hohlraums (32) des menschlichen oder tierischen Körpers anzupassen, wenn er aufgeblasen ist.

7. Aufblasbarer Ballon (10) zur medizinischen Verwendung nach einem der Ansprüche 1 bis 6, umfassend außerdem einen entfernbaren Einführungsstift (36), der sich nach außen hin in der Verlängerung eines Stutzens der Aufblasöffnung (22) erstreckt, und einen entfernbaren Draht (38) zur Anbringung, der sich entlang der Sohle (30) oder in dieser Letzteren erstreckt.

## Claims

1. Inflatable balloon (10) for medical use, intended to be inserted deflated into a human or animal body cavity and then inflated once inside the cavity so as to apply pressure against it, comprising:
- a balloon body (12), the wall (14) of which is made from a flexible inflatable material, with a generally elongated shape having a proximal inflation end (16) and a distal positioning end (18) at the back of the cavity, wherein the wall (14) further locally has an elongated portion (30) called the sole that is stiffer than the rest of the wall (14), this sole extending from the proximal end (16) to the distal end (18),
- an inflation opening (22) formed in the proximal end (16) through which a fluid is introduced into the balloon body (12) so as to inflate it under pressure, and
- an endoscope opening (24) further formed in the proximal end (16), this endoscope opening (24) being designed for the introduction of an endoscope into the balloon body (12),
**characterized in that** the endoscope opening (24) leads into the inside of the balloon body (12) at the inside of an elongated sock (26) that has a closed distal end (28) not fixed to the wall (14) of the balloon body (12).

2. Inflatable balloon (10) for medical use according to claim 1, wherein the sole (30) is an add-on part in contact with the wall (14) of the balloon body (12).

3. Inflatable balloon (10) for medical use according to claim 1, wherein the sole (30) is made of the same material as the rest of the wall (14) of the balloon body (12), but it is significantly thicker than the rest of this wall (14).

4. Inflatable balloon (10) for medical use according to any one of claims 1 to 3, further comprising a stiffener (40) at the distal end prolonging the sole (30), this distal stiffener (40) also being stiffer that the rest of the wall (14) of the balloon body (12) and being shaped so as to prevent any elongation of the balloon (10) during its inflation, in cooperation with the sole (30).

5. Inflatable balloon (10) for medical use according to any one of claims 1 to 4, wherein the endoscope opening (24) comprises a valve for the insertion of an endoscope while preventing any outlet of fluid.

6. Inflatable balloon (10) for medical use according to any one of claims 1 to 5, for use in otorhinolaryngology, wherein the balloon body (12) is preformed to match the internal shapes of a nasal cavity (32) of a human or animal body when it is inflated.

7. Inflatable balloon (10) for medical use according to any one of claims 1 to 6, further comprising a removable insertion rod (36) extending outwards along the prolongation of an end piece of the inflation opening (22) and a removal placement wire (38) extending along the sole (30) or inside the sole.
